# EUROPÄISCHE PATENTSCHRIFT

(11) **EP 2 171 036 B1**
(45) Veröffentlichungstag und Bekanntmachung des Hinweises auf die Patenterteilung: **04.04.2018**
(21) Anmeldenummer: 08784382.7
(22) Anmeldetag: 24.07.2008
(51) Int. Cl.: C12M 1/22, C12M 1/20

(54) **VERFAHREN UND VORRICHTUNG ZUR KULTIVIERUNG LEBENDER ZELLEN**
METHOD AND APPARATUS FOR CULTIVATING LIVING CELLS
PROCÉDÉ ET DISPOSITIF POUR LA CULTURE DE CELLULES VIVANTES

(30) Priorität: 02.08.2007 DE 102007036611
(43) Veröffentlichungstag der Anmeldung: 07.04.2010
(73) Patentinhaber: Deutsche Diabetes-Forschungsgesellschaft e. V., 40225 Düsseldorf (DE)
(72) Erfinder: BURKART, Volker, 40225 Düsseldorf (DE); BURKART, Thomas, 40225 Düsseldorf (DE); BURKART, Andreas, 40225 Düsseldorf (DE)
(74) Vertreter: Schrooten, Rolf
(86) Internationale Anmeldenummer: PCT/DE2008/001206
(87) Internationale Veröffentlichungsnummer: WO 2009/018799

(56) Entgegenhaltungen:
- EP-A- 0 282 840
- WO-A-00/17315
- WO-A-00/72968
- WO-A-2006/037022
- DE-B4-102007 036 611
- US-A- 5 508 197
- US-A- 5 578 490
- US-A- 5 726 060
- US-B1- 6 794 184

## Beschreibung

### Erfindungsgebiet

Die Erfindung betrifft ein Verfahren zur Kultivierung lebender Zellen in einer Flüssigkeit, wobei die Zellen in mindestens einen Kulturraum eingebracht werden, der mit mindestens einem Reservoir für die Flüssigkeit in Verbindung steht, wobei das Volumen der Flüssigkeit in mindestens einem Kulturraum konstant gehalten wird. Die Erfindung betrifft ferner eine Vorrichtung zur Kultivierung lebender Zellen in einer Flüssigkeit, die mindestens einen Kulturraum umfasst, der über mindestens ein Verbindungselement mit mindestens einem Reservoir zur Aufnahme der Flüssigkeit verbunden ist, wobei das Verbindungselement derart ausgebildet ist, dass das Volumen der Flüssigkeit in mindestens einem Kulturraum konstant gehalten wird. Die Erfindung betrifft auch Verwendungen dieser Vorrichtung.

### Hintergrund der Erfindung

Isolierte Zellen, Zellpopulationen oder Organe bis hin zu intakten Individuen wie Mäusen und Menschen stellen komplexe biologische System dar, deren einzelne Komponenten in vielfältiger Weise miteinander kommunizieren. Diese Kommunikation erfolgt im Wesentlichen über sogenannte "Mediatoren", die man auch als "Botenstoffe" oder "lösliche Botenstoffe" bezeichnen kann. Zu diesen Botenstoffen zählen beispielsweise Hormone oder bestimmte Gruppen von Proteinen wie Zytokine oder Chemokine, die primär von Immunzellen gebildet werden. Im weiteren Sinne könnte man auch Immunglobuline (Antikörper) zu dieser Substanzgruppe zählen. Von Zellen oder Geweben freigesetzte Botenstoffe haben eine gewisse "Fernwirkung", das heißt, sie wirken nicht nur in unmittelbarer Nähe der Zelle, von der sie freigesetzt werden, sondern sie können systemisch ganze Organe oder den gesamten Körper beeinflussen. Durch diese "Fernwirkung" tragen die Botenstoffe somit beispielsweise zum kontrollierten Ablauf von Immunreaktionen bei oder sie regeln die Reaktion von Zellen oder Organsystemen auf Änderungen der Stoffwechsellage. Da die Botenstoffe relativ stabil (langlebig) sind, können sie sich in Zellkulturüberständen (oder auch im Serum von Tieren, Patienten) anreichern, wo sie dann auch mit entsprechenden spezifischen und empfindlichen Nachweisverfahren quantifiziert werden können. Durch die Einwirkung von pharmakologischen Substanzen, im Verlauf von Entzündungen oder in bestimmten Stoffwechselsituationen kann die Freisetzung dieser Botenstoffe entscheidend beeinflusst werden.

In zunehmendem Maße erlangt die Isolation kleiner und kleinster, zum Teil hoch spezialisierter Zellpopulationen aus tierischem und humanem Probenmaterial, beispielsweise Populationen von Immunzellen und endokrinen Zellen, und deren Einsatz zu Forschungszwecken größere Bedeutung. Die Langzeit-Beobachtung der Funktionen dieser empfindlichen Primär-Zellpopulationen *in vitro* erfordert häufig die vielfache Entnahme von Aliquots des Kulturüberstandes zur Bestimmung freigesetzter Mediatoren. Die Langzeitkultur unter verschiedenen experimentellen Bedingungen stellt dabei hohe Ansprüche an möglichst schonende Kultivierungsverfahren. Zurzeit werden Experimente mit geringen Zellzahlen überwiegend in Petrischalen mit 2 bis 5 ml Volumen oder in den Vertiefungen von Multiwellplatten, beispielsweise 1/2 area 96-Well Mikrotiterplatten mit 100 µl Volumen, angesetzt.

Die Kultivierung in Petrischalen hat aber den Nachteil, dass hierbei ein hoher Bedarf an Substanzen besteht, die zur Modulation der Zellfunktionen eingesetzt werden müssen, was zu sehr hohen Kosten führt. Darüber hinaus werden die von den Zellen freigesetzten Mediatoren sehr stark verdünnt, so dass ein Nachweis der Mediatoren im Kulturüberstand nicht oder nur schwer möglich ist. Die Kultivierung in Petrischalen hat ferner den Nachteil, dass sehr starke Schwankungen zwischen den Ansätzen in verschiedenen Petrischalen innerhalb eines Ansatzes auftreten können, was die Reproduzierbarkeit der Ergebnisse stark beeinträchtigt. Darüber hinaus führen fehlende oder geringe Zell-Zell-Kontakte zu einer Verfälschung der Zellreaktionen.

Aber auch die Kultivierung in Mikrotiterplatten ist mit erheblichen Nachteilen verbunden. So erfordert die wiederholte Entnahme von Aliquots des Kulturmediums aus den Wells zur Messung freigesetzter Mediatoren jeweils die Zugabe von frischem Medium zum Volumenausgleich. Dadurch können sich, beispielsweise durch die Addition von Pipettierfehlern über die Versuchsdauer große Volumenunterschiede zwischen den einzelnen Vertiefungen ergeben. Ferner können sich das über den Zellen in den Vertiefungen verbliebene Medium und die zum Volumenausgleich zugegebenen Medium-aliquots in Temperatur, pH-Wert, p0₂ usw. erheblich unterscheiden. Dadurch tritt bei jeder erneuten Zugabe von Medium eine Irritation der kultivierten Zellen auf, was die Kontinuität der Kulturbedingungen stört.

Aus der WO-A-00 729 68 ist eine Vorrichtung zur Behandlung von flüssigen Proben bekannt, die im Wesentlichen aus drei übereinander angeordneten Platten besteht, welche jeweils über Kanäle miteinander verbunden sind. Die oberste Platte weist Vertiefungen auf, die als Reservoir für die Reagenzien dienen. Die mittlere Platte umfasst kleine Reservoirs, Kanäle und Rückstrom-Ventile, die der Verteilung der Reagenzien auf die Reaktionsräume der unteren Platte dienen. Die unterste Platte weist eine Vielzahl von Reaktionsräumen auf und kann beispielsweise eine 96-well oder 384-well Mikrotiterplatte sein. Die gleichmäßige Verteilung der Reagenzien von der oberen Platte auf die Reaktionsräume der unteren Platte erfolgt mittels eines druckbetriebenen Zuführsystems, das gleichzeitig ein Entleeren oder Waschen der Reaktionsräume ermöglicht. Diese Anordnung-der Platten dient dazu, die Reaktionsräume der unteren Platte gleichzeitig und gleichmäßig, d. h. mit gleichen Volumina, zu befüllen. Dabei werden die Reaktionsräume der unteren Platte mit den Reagenzien gefüllt, anschließend die gewünschte Analyse bzw. Reaktion durchgeführt und dann die Reaktionsräume gewaschen.

Die EP-A-0 282 840 offenbart eine Vorrichtung für chemische und biochemische Analysen sowie die Identifikation von Mikroorganismen, die eine Vielzahl von Reaktionsräumen aufweist. Die Vorrichtung umfasst ferner eine Verteileinrichtung zum Befüllen der Reaktionsräume und ein Entlüftungssystem, durch das die aus den Reaktionsräumen verdrängte Luft entweichen kann. Es ist ferner ein Reservoir vorgesehen, aus dem eine Flüssigkeit durch die Verteileinrichtung in die Reaktionsräume fließen kann. Die Flüssigkeit fließt dabei drucklos durch die Vorrichtung, wobei durch die Position der Entlüftungsleitung, die oberhalb der Flüssigkeitsoberfläche in dem Reservoir angeordnet ist, sichergestellt wird, dass die Reaktionsräume gefüllt werden.

Aus der US-A-5 578 490 ist beispielsweise eine Zellkulturplatte bekannt, bei der die eigentlichen Kulturräume jeweils durch eine Membran von einem dem jeweiligen Kulturraum zugeordneten Reservoir getrennt sind. Die Membran ist für im Zellkulturmedium gelöste Substanzen durchlässig, hält aber die Zellen im Kulturraum zurück. Es können also sowohl gelöste Substanzen wie beispielsweise Nährstoffe aus dem Reservoir in den Kulturraum als auch gelöste Substanzen wie beispielsweise Metaboliten aus dem Kulturraum in das Reservoir diffundieren. Diese Zellkulturplatte hat daher den Nachteil, dass auch zu bestimmende Mediatoren aus dem Kulturraum heraus diffundieren, so dass die Konzentration der von den Zellen freigesetzten Mediatoren sehr stark verringert wird. Folglich ist hier ein Nachwels von Mediatoren im Kulturüberstand nicht oder nur schwer möglich.

Aus der US-A-5 726 060 ist ferner ein Verfahren zur Kultivierung respiratorischer Epithelzellen aus Säugetieren bekannt, bei dem die Zellen in einem Einsatz kultiviert werden, der einen für Flüssigkeiten durchlässigen Boden aufweist, auf dem die Zellen adhärent wachsen können. Der Einsatz wird in eine Kulturschale gestellt, die als Reservoir für das Kulturmedium dient. Durch Austauschen des Mediums in der Kulturschale können die Zellen so mit frischem Medium versorgt werden, ohne dass sie hierdurch geschädigt oder irritiert werden. Allerdings hat auch dieses Verfahren den Nachteil, dass zu bestimmende Mediatoren aus dem Einsatz heraus diffundieren können, so dass die Konzentration der von den Zellen freigesetzten Mediatoren sehr stark verringert wird und somit ein Nachweis von Mediatoren im Kulturüberstand nicht oder nur schwer möglich ist.

### Zusammenfassung der Erfindung

Es ist Aufgabe der Erfindung die genannten Nachteile zu vermeiden und ein Verfahren und eine Vorrichtung der eingangs genannten Art zur Verfügung zu stellen, mit denen auch kleine Gewebeproben und Zellen in geringen Zellzahlen über einen längeren Zeitraum kultiviert werden können, ohne dass die Konzentration der von den Zellen freigesetzten Mediatoren durch Verdünnung oder Diffusion wesentlich verringert wird.

Erfindungsgemäß wird die Aufgabe durch ein Verfahren der eingangs genannten Art gelöst, bei dem das Volumen der Flüssigkeit über einen ventilartigen Mechanismus konstant gehalten wird, welcher gleichzeitig einen Übertritt von Flüssigkeit und/oder in der Flüssigkeit gelösten oder suspendierten Substanzen aus dem Kulturraum in das Reservoir verhindert. Erfindungsgemäß muss also im Kulturraum keine Flüssigkeit bzw. Medium nachgefüllt oder gewechselt werden, da immer ausreichend Flüssigkeit aus dem Reservoir über den ventilartigen Mechanismus in den Kulturraum strömen kann. Durch die gleichförmige Flüssigkeitszufuhr in alle Kulturräume (Replikate) aus mindestens einem Reservoir werden optimale Bedingungen für eine Langzeitkultur geschaffen. In dem erfindungsgemäßen Verfahren werden Zellen und/oder Gewebe beispielsweise in einem Kulturraum mit Kulturmedium oder ähnlichen Nährflüssigkeiten, vorzugsweise in einem kleinen Volumen (ca. 100 µl), inkubiert. Der Kulturraum ist mit einem Reservoir verbunden, das vorzugsweise ein größeres Volumen des Kulturmediums enthält (>5 ml), so dass ein kontinuierlicher Übertritt von Flüssigkeit aus dem Reservoir in den Kulturraum zum kontinuierlichen Volumenausgleich ermöglicht wird, wobei ein gleichmäßiger Volumenausgleich in allen Kulturräumen eines Versuchsansatzes erfolgt. Durch das Konstanthalten des Volumens im Kulturraum wird die Kultur kleiner Gewebeproben oder geringer Zellzahlen in kleinem Volumen auch über einen längeren Zeitraum möglich, was wiederum zu einer Verbesserung der Zell-Zell-Kontakte, einer stärkeren Anreicherung freigesetzter Mediatoren und einem geringeren Bedarf an Substanzen zur Modulation der Zell-/Gewebe-Funktionen führt. Mittels des erfindungsgemäßen Verfahrens kann also die Freisetzung von Mediatoren bzw. Botenstoffen zeitabhängig unter den Einflüssen verschiedener experimenteller Bedingungen (z.B. Einsatz von pharmakologischen Substanzen) exakt geprüft werden. Erfindungsgemäß wird ein Übertritt von Flüssigkeit und/oder in der Flüssigkeit gelösten oder suspendierten Substanzen aus dem Kulturraum in das Reservoir durch den ventilartigen Mechanismus verhindert, wodurch ein Verlust von Mediatoren und somit eine Verringerung der Konzentration dieser Botenstoffe vermieden werden kann. Insgesamt wird hierdurch in vorteilhafter Weise eine Verdünnung oder Verfälschung der Konzentrationen der durch die Zellen freigesetzten Mediatoren verhindert, was die spätere Analyse deutlich vereinfacht oder überhaupt erst möglich macht. Der ventilartige Mechanismus ermöglicht also einerseits einen Übertritt von Flüssigkeit bzw. Kulturmedium aus dem Reservoir in den Kulturraum zum Volumenausgleich, verhindert aber andererseits einen Übertritt von Medium (und darin gelösten Modulatoren, Mediatoren usw.) aus dem Kulturraum in das Reservoir auf sehr effektive Weise.

Vorzugsweise verschließt innerhalb des ventilartigen Mechanismus' zumindest ein Verschlusskörper aufgrund seines Eigengewichts eine Öffnung und/oder einen Kanal, so dass keine Flüssigkeit aus dem Kulturraum in das Reservoir entweichen kann. Gleichzeitig ermöglicht der Verschlusskörper aber einen Volumenausgleich im Kulturraum, wenn diesem Flüssigkeit entnommen oder durch Verdunstung entzogen wird, da bei einer Verringerung des Drucks durch die Flüssigkeitssäule im Kulturraum Flüssigkeit aus dem Reservoir in den Kulturraum strömen kann.

Es ist besonders von Vorteil, wenn das Reservoir in unmittelbarer Nähe des Kulturraums angeordnet wird, da in diesem Fall der Volumenausgleich im Kulturraum mit Flüssigkeit aus dem benachbarten Reservoir mit angeglichenem pH-Wert, gleicher Temperatur, angeglichenem Sauerstoffpartialdruck (p0₂) etc. erfolgen kann. Darüber hinaus ergibt sich durch die direkte Nachbarschaft des Reservoirs ein verbesserter Verdunstungsschutz im Kulturraum. Bei Standard-Mikrotiter-/Multiwell-Platten sind die Verdunstungsverluste in den am Rande liegenden Wells (trotz aufliegendem Deckel) am stärksten. Durch die besonders vorteilhafte Nähe des Reservoirs zu den Kulturräumen, bei mehreren Kulturräumen vorzugsweise im zentralen Bereich der erfindungsgemäßen Vorrichtung, sind die Kulturräume erfindungsgemäß vorzugsweise vollständig von dem mit Flüssigkeit gefüllten Reservoir umgeben. Die im Vergleich zu den Kulturräumen sehr große Flüssigkeitsoberfläche des Reservoirs hält die Verdunstungsverluste aus den zentral gelegenen Kulturräumen bei Ausführungsformen mit mehreren Kulturräumen somit äußerst gering.

Insbesondere bei der Kultivierung von Zellen, die nur in sehr geringen Mengen verfügbar sind, ist es von Vorteil, wenn der Kulturraums mit einem geringen Volumen Flüssigkeit gefüllt wird, vorzugsweise maximal 200 µl, besonders bevorzugt maximal 100 µl oder 50 µl.

In besonders vorteilhafter Ausgestaltung des erfindungsgemäßen Verfahrens ist ferner vorgesehen, dass das Reservoir mit einem Volumen gefüllt wird, das mindestens das 5-fache, vorzugsweise 10-fache, besonders bevorzugt 100-fache, des Volumens der Flüssigkeit im Kulturraum beträgt. Dadurch, dass das Volumen im Reservoir deutlich größer als in einem Kulturraum oder der Summe aller Kulturräume ist, ist ein kontinuierlicher Volumenausgleich gewährleistet, ohne dass sich das Volumen im Kulturraums oder in den Kulturräumen merklich verringert.

Die Aufgabe wird erfindungsgemäß ferner durch eine Vorrichtung der eingangs genannten Art gelöst, bei der das Verbindungselement einen ventilartigen Mechanismus umfasst. Durch eine solche Ausbildung des Verbindungselements wird eine gleichförmige Flüssigkeitszufuhr in den Kulturraum bzw. die Kulturräume (Replikaten) aus einem Reservoir ermöglicht, um optimale Bedingungen für eine Langzeitkultur zu schaffen. Das Verbindungselement ermöglicht also in vorteilhafter Weise einen Übertritt von Flüssigkeit aus dem Reservoir in mindestens einen Kulturraum zum Volumenausgleich. Durch das Konstanthalten des Volumens im Kulturraum wird die Kultur kleiner Gewebeproben oder geringer Zellzahlen in kleinem Volumen auch über einen längeren Zeitraum möglich, was wiederum zu einer Verbesserung der Zell-Zell-Kontakte, einer Vermeidung der Verdünnung oder Verfälschung der Konzentrationen der durch die Zellen freigesetzten Mediatoren und einem geringeren Bedarf an Substanzen zur Modulation der Zell-/Gewebe-Funktionen führt.

Der ventilartige Mechanismus ermöglicht in vorteilhafter Weise einen Übertritt von Flüssigkeit aus dem Reservoir in mindestens einen Kulturraum. Gleichzeitig verhindert der Mechanismus aber auch einen Übertritt von Flüssigkeit und vor allem darin gelösten Modulatoren, Mediatoren und Ähnlichem aus dem Kulturraum in das Reservoir. Folglich reichern sich potentielle Zielmoleküle für eine spätere Analyse an, was insbesondere bei geringen Zellzahlen von besonderem Vorteil ist. Ein weiterer Vorteil des erfindungsgemäßen ventilartigen Mechanismus' gegenüber den bekannten Lösungen mit Membranen liegt darin, dass die "Ventil-Lösung" auch den Übertritt von hochmolekularen Substanzen aus dem Reservoir in die jeweiligen Kulturräume zulässt. Membranen besitzen dagegen in der Regel eine "molekulare Ausschlussgrenze", die den Übertritt von hochmolekularen Substanzen, wie beispielsweise Serumproteinen, verhindert. Serumproteine sind wichtige Komponenten von Kulturmedien und für die Kultivierung der meisten Säugetier-Zellen unbedingt erforderlich. Beim Einsatz von Membranen können somit diese wichtigen Mediumskomponenten nicht vollständig oder nur verzögert vom Reservoir in den Kulturraum übertreten, wodurch die kultivierten Zellen geschädigt würden. Weiterhin können durch Ladungen an der Oberfläche des Membranmaterials (kationische oder anionische Materialien) geladene Bestandteile des Kulturmediums (z.B. bestimmte Aminosäuren) an der Passage durch die Membran gehindert werden. "Ventile" aus zellkulturgeeigneten Kunststoffen, deren Oberflächen normalerweise keine Ladungen aufweisen, besitzen diese negativen Eigenschaften nicht.

In besonders vorteilhafter Ausgestaltung der erfindungsgemäßen Vorrichtung ist vorgesehen, dass das Verbindungselement mindestens eine Öffnung und/oder mindestens einen Kanal umfasst, so dass nur ein begrenzter, gut kontrollierbarer Bereich zum Übertritt der Flüssigkeit zur Verfügung steht.

In weiterer vorteilhafter Ausgestaltung der erfindungsgemäßen Vorrichtung ist vorgesehen, dass der ventilartige Mechanismus zumindest einen Verschlusskörper umfasst. Dieser Verschlusskörper kann beispielsweise kugel-, zylinder-, kegel- oder klappenförmig ausgebildet sein.

Der Verschlusskörper kann in vorteilhafter Ausgestaltung der Erfindung auch eine Folie sein. Die Folie kann beispielsweise in dem Verbindungselement durch Kleben oder Schweißen befestigt sein. Alternativ kann die Folie auch in einen Rahmen oder Ähnliches eingespannt sein, wobei bei dieser Ausführungsform vorzugsweise der Rahmen in dem Verbindungselement befestigt ist. In jedem Fall ist die Folie frei beweglich befestigt, so dass sie in Abhängigkeit von den jeweils herrschenden Druckverhältnissen den Kanal des Verbindungselements entweder verschließt oder frei gibt. Die Folie kann beispielsweise aus Kunststoff oder einem imprägnierten textilen Material bestehen. In jedem Fall ist das Material so zu wählen, dass die korrekte Funktion des Verschlusskörpers gewährleistet ist. Dabei spielen vor allem Gewicht, Elastizität sowie Kleb- oder Schweißbarkeit des Materials eine wichtige Rolle.

In einer besonderen Ausführungsform sind eine Vielzahl von Kulturräumen vorgesehen, vorzugsweise 8, 16 oder 32. Die erfindungsgemäße Vorrichtung kann also auch nach Art einer Multiwellplatte bzw. Mikrotiterplatte ausgebildet sein, was in vorteilhafter Weise eine Anwendung in Reihenversuchen und in Hochdurchsatzverfahren (High Throughput Screening) ermöglicht. Dabei sind die Kulturräume vorzugsweise voneinander getrennt, wobei jeder einzelne Kultur raum ein separates Verbindungselement aufweist, so dass der Volumenausgleich in jedem Kulturraum erfolgen kann, ohne dass Kreuzkontaminationen auftreten können.

Wenn die erfindungsgemäße Vorrichtung zumindest teilweise, beispielsweise im Bereich des Bodens, aus klarem Kunststoff besteht, ist eine kontinuierliche mikroskopische Beobachtung der kultivierten Zellen/Gewebe sowie eine Auswertung von zell-/ gewebespezifischen Parametem mit Hilfe colorimetrischer/photometrischer Verfahren (Mehrkanal Photometer) möglich. Bei einer Ausführungsform der erfindungsgemäßen Vorrichtung mit mehreren Kulturräumen im Format einer Standard-Mikrotiter-/Multiwell-Platte ist ferner die Zentrifugation mit speziellen Zentrifugenrotoren (-Einsätzen) möglich, die von vielen Herstellern von Laborzentrifugen angeboten werden, in den meisten Zell-/Gewebekulturlabors vorhanden sind und routinemäßig eingesetzt werden.

Die erfindungsgemäße Vorrichtung kann zur Langzeit-Kultivierung von lebenden Zellen oder Geweben in geringen Mengen und/oder geringem Volumen, insbesondere in Mehrfachansätzen für kinetische Studien unter verschiedenen experimentellen Bedingungen, verwendet werden. Damit ist die Vorrichtung, wie auch das erfindungsgemäße Verfahren, für die Kultivierung und Beobachtung einer Vielzahl von Zelltypen (primäre Zellen, Zelllinien) aus dem Menschen und aus Tieren (z.B. Maus, Ratte) geeignet, die nur in geringer Zahl auftreten oder nur in geringen Mengen isoliert werden können, wie beispielsweise:
- Endokrine Zellen: Zellen der Nebenniere, Insulin-produzierende Betazellen aus der Bauchspeicheldrüse;
- Immunzellsubpopulationen: Regulatorische T-Lymphozyten, Natürliche Killerzellen;
- Endothelzellen von Blutgefäßen;
- Vorläuferzellen für unterschiedliche Gewebe: Knochen, Lungenepithel; oder
- Nervenzellen.

Die erfindungsgemäße Vorrichtung kann ferner zum Screening von Substanzen verwendet werden, insbesondere Substanzen, die das Überleben und/oder die Funktion (Freisetzung von Mediatoren wie Hormone, immunologische Botenstoffe (Zytokine)) von Zellen und Geweben beeinflussen (Toxizitätstests, Prüfung pharmakologischer Inhibitoren/Stimulatoren). Das Kultursystem erleichtert insbesondere Verlaufsanalysen zur Ermittlung des Zeitpunktes zu dem beispielsweise die Wirkung einer bestimmten Substanz eintritt oder ihr Optimum erreicht.

Die Erfindung wird im Folgenden anhand der folgenden Abbildungen beispielhaft näher erläutert.

### Kurze Beschreibung der Abbildungen

- Figur 1: zeigt eine schematische Seitenansicht (Längsschnitt) einer Ausführungsform der erfindungsgemäßen Vorrichtung mit einem Reservoir mit ungefähr 5 ml Volumen und einem Kulturraum mit ungefähr 100 µl Volumen.
- Figur 2: zeigt eine schematische Seitenansicht (Längsschnitt) einer weiteren Ausführungsform der erfindungsgemäßen Vorrichtung mit einem ventilartigen Mechanismus innerhalb des Verbindungselements.
- Figur 3: zeigt eine perspektivische Ansicht des ventilartigen Mechanismus' gemäß Figur 2.
- Figuren 4-8: zeigen Schnitte durch verschiedene Ausführungsformen der Verbindungselemente mit Ansichten von der Seite und nach Drehung um 90° von vorne, a) Seitenansicht, b) Vorderansicht.
- Figur 9: zeigt eine Ausführungsform der erfindungsgemäßen Vorrichtung mit 16 Kulturräumen.

### Beschreibung beispielhafter und bevorzugter Ausführungsformen der Erfindung

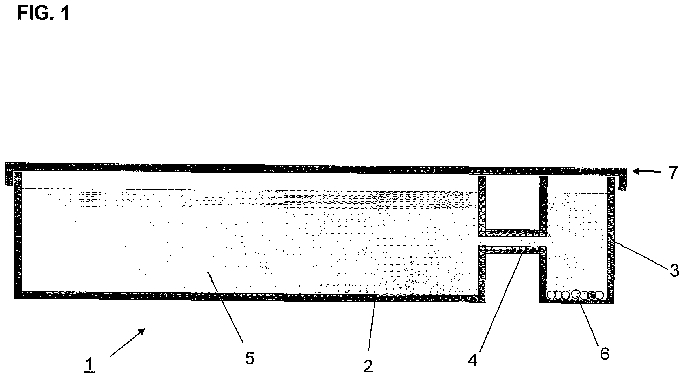

Figur 1 zeigt eine schematische Seitenansicht einer Ausführungsform einer erfindungsgemäßen Vorrichtung 1 mit einem Reservoir 2 und einem Kulturraum 3. Das Reservoir 2 ist mit dem Kulturraum 3 über ein Verbindungselement 4 verbunden. Das Verbindungselement 4 weist eine hier nicht dargestellte Einrichtung oder einen Mechanismus auf, die bzw. der verhindert, dass eine sich im Kulturraum 3 befindliche Flüssigkeit 5, bei der es sich beispielsweise um ein Kulturmedium für lebende Zellen oder eine sonstige Nährlösung handeln kann, aus dem Kulturraum 3 in das Reservoir 2 strömt. Gleichzeitig erlaubt die Einrichtung aber einen Übertritt der Flüssigkeit 5 aus dem Reservoir 2 in den Kulturraum 3. Auf diese Weise kann ein Volumenausgleich in dem Kulturraum 3 stattfinden, wenn diesem Flüssigkeit 5 entnommen oder beispielsweise durch Verdunstung entzogen wird. Das heißt, es findet automatisch und kontinuierlich ein Angleichen der Höhe des Flüssigkeitsspiegels zwischen dem Reservoir 2 und dem Kulturraum 3 statt. Sobald der Flüssigkeitsspiegel im Kulturraum 3 absinkt, strömt Flüssigkeit 5 solange aus dem Reservoir 2 in den Kulturraum 3 bis die Flüssigkeitsspiegel in beiden Kompartimenten wieder die gleiche Höhe einnehmen. Da im hier dargestellten Ausführungsbeispiel das Volumen der Flüssigkeit 5 im Reservoir 2 (5 ml) deutlich größer, d. h. hier 50 Mal größer, als im Kutturraum 3 (100 µl) ist, bleibt das Volumen der Flüssigkeit 5 im Kulturraum praktisch konstant. Dadurch, dass im hier dargestellten Ausführungsbeispiel das Reservoir 2 in unmittelbarer Nähe des Kulturraums 3 angeordnet ist, d. h. diese nur über ein kurzes Verbindungselement 4 verbunden sind, kann der Volumenausgleich im Kulturraum 3 mit Flüssigkeit 5 aus dem benachbarten Reservoir 2 mit angeglichenem pH-Wert, gleicher Temperatur, angeglichenem Sauerstoffpartialdruck (p0₂) usw. erfolgen. Hierdurch werden die Kulturbedingungen konstant gehalten und unnötiger Stress für die Zellen 6 bzw. Gewebe im Kulturraum 3 vermieden. Darüber hinaus ergibt sich durch die direkte Nachbarschaft des Reservoirs 2 ein verbesserter Verdunstungsschutz im Kulturraum 3. Gemäß dem erfindungsgemäßen Verfahren kann die hier dargestellte Vorrichtung 1 beispielsweise zur Kultivierung von lebenden Zellen 6 in geringer Anzahl und in einem kleinen Volumen verwendet werden. Die Zellen 6 werden zu diesem Zweck beispielsweise in 50 µl Medium aufgenommen und dann in den Kulturraum 3 eingebracht. Nach der Sedimentation oder dem Abzentrifugieren der Zellen 6 befinden sich diese überwiegend am Boden des Kulturraums 3. Bei einer Ausführungsform der erfindungsgemäßen Vorrichtung mit mehreren Kulturräumen im Format einer Standard-Mikrotiter-/Multiwell-Platte ist auch die Zentrifugation mit speziellen Zentrifugerirotoren (-Einsätzen) möglich, die von vielen Herstellern von Laborzentrifugen angeboten werden, in den meisten Zell-/Gewebekulturlabors vorhanden sind und routinemäßig eingesetzt werden. Nun kann ein größeres Volumen Medium, beispielsweise 5 oder 10 ml, in das Reservoir 2 eingefüllt werden, woraufhin der Flüssigkeitsspiegel im Kulturraum 3 bis auf das Niveau des Reservoirs 2 steigt. Die Zellkultur kann jetzt über einen längeren Zeitraum unter konstanten Bedingungen gehalten werden, wobei das Volumen im Kulturraum 3 annähernd konstant bleibt, auch wenn Flüssigkeit zur Testzwecken entnommen wird.

Die erfindungsgemäße Vorrichtung 1 ist in diesem Ausführungsbeispiel wie eine eine Standard-Mikrotiter-/Multiwell-Platte mit einem abnehmbaren Deckel 7 aus transparentem Kunststoff abgedeckt. Dieser Deckel 7 ist primär wichtig, um die Sterilität des Systems über die Dauer der Kultur zu gewährleisten. Er liegt nicht dicht auf der Vorrichtung 1 auf, sondern ermöglicht, wie der Deckel einer Petrischale, durch kleine Abstandshalter (hier nicht dargestellt) den für die Stabilisierung des pH-Wertes wichtigen Gasaustausch zwischen dem Kultursystem und dem Innenraum eines Inkubators.

Bei Standard-Mikrotiter-/Multiwell-Platten sind die Verdunstungsverluste in den am Rande liegenden Wells (trotz aufliegendem Deckel) am stärksten. Durch die besonders vorteilhafte Nähe des Reservoirs zu den Kulturräumen, bei mehreren Kulturräumen vorzugsweise im zentralen Bereich der erfindungsgemäßen Vorrichtung, sind die Kulturräume idealerweise vollständig von dem mit Flüssigkeit gefüllten Reservoir umgeben. Die im Vergleich zu den Kulturräumen sehr große Flüssigkeitsoberfläche des Reservoirs hält die Verdunstungsverluste aus den zentral gelegenen Kulturräumen bei Ausführungsformen mit mehreren Kulturräumen somit äußerst gering.

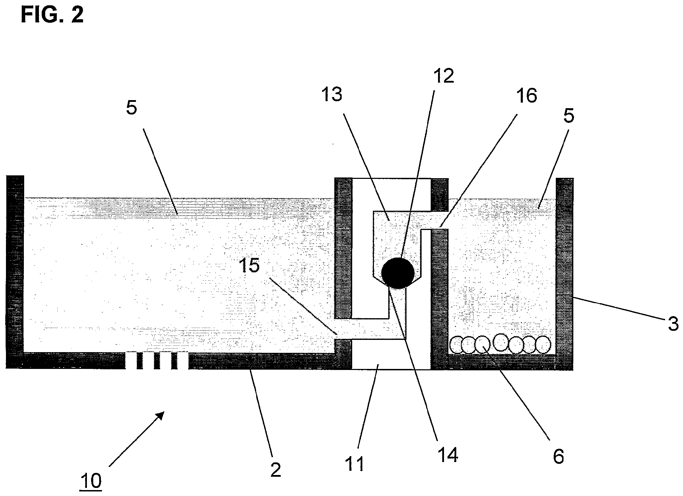

Figur 2 zeigt eine schematische Seitenansicht einer weiteren Ausführungsform einer erfindungsgemäßen Vorrichtung 10 mit einem ventilartigen Mechanismus innerhalb eines Verbindungselements 11. Der ventilartige Mechanismus umfasst einen zylinderförmigen Verschlusskörper 12, der innerhalb des Kanals 13, der das Reservoir 2 mit dem Kulturraum 3 verbindet, angeordnet ist. Der ventilartige Mechanismus in diesem Ausführungsbeispiel basiert auf dem technischen Prinzip der "eigengewichtgesteuerten (Rückschlag-)Ventile" und ermöglicht einen gerichteten Fluss der Flüssigkeit 5 vom Reservoir 2 in den Kulturraum 3.

Der erfindungsgemäße ventilartige Mechanismus weist dabei die folgenden Vorteile auf:
- hohe Funktionssicherheit,
- einfache Herstellung in kleinstem Maßstab ist möglich,
- die Verwendung zell-/gewebekulturtauglicher Kunststoffe ist möglich,
- es tritt keine Beeinträchtigung der Funktion während des Transports oder der Lagerung der Kultursysteme auf.

Der Verschlusskörper 12 verschließt durch sein Eigengewicht die Öffnung 14 im Kanal 13, so dass keine Flüssigkeit (einschließlich der darin gelösten Stoffe) aus dem Kulturraum 3 in das Reservoir 2 fließen kann. Sobald aber der Druck der Flüssigkeitssäule im Reservoir 2 größer wird als der Druck der Flüssigkeitssäule im Kulturraum 3, beispielsweise bei Entnahme von Flüssigkeit aus dem Kulturraum 3, wird der Verschlusskörper 12 nach oben gedrückt, so dass er die Öffnung 14 freigibt. Flüssigkeit 5 kann dann aus dem Reservoir 2 in den Kulturraum strömen, bis sich die Flüssigkeitsspiegel in beiden Kompartimenten wieder angeglichen haben. Der Verschlusskörper 12 sinkt dann aufgrund seines Eigengewichts wieder nach unten und verschließt die Öffnung 14, so dass keine Flüssigkeit 5 aus dem Kulturraum 3 in das Reservoir 2 zurückfließen kann. Der erfindungsgemäße Mechanismus ist sehr effektiv und gewährleistet einen kontinuierlichen Volumenausgleich im Kulturraum 3, ohne dass durch die Zellen 6 produzierte und im Kulturüberstand nachzuweisende Stoffe verdünnt werden oder entweichen können. Im vorliegenden Ausführungsbeispiel ist der Kanal 13 des Verbindungselements 11 derart angeordnet, dass die Eintrittsöffnung 15, durch die die Flüssigkeit 5 aus dem Reservoir 2 in den Kanal 13 strömt, in der Nähe des Bodens des Reservoirs 2 liegt, während die Austrittsöffnung 16, durch die die Flüssigkeit 5 aus dem Kanal 13 in den Kulturraum 3 fließt, im oberen Bereich des Kulturraums 3 liegt. Diese Anordnung hat den Vorteil, dass die Zellen 6 nicht durch einströmende Flüssigkeit 5 irritiert werden. Die Zellen 6 können also in der erfindungsgemäßen Vorrichtung 10 ungestört über einen längeren Zeitraum in einem kleinen Volumen kultiviert werden.

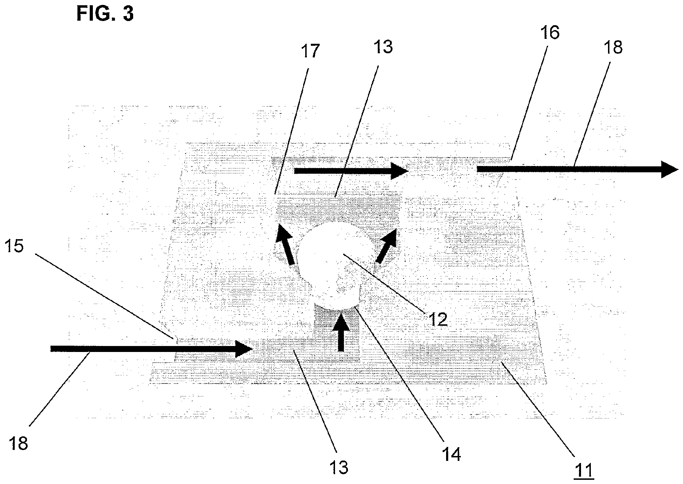

Figur 3 zeigt eine perspektivische Ansicht des Verbindungselements 11 der Vorrichtung 10 gemäß Figur 2. Es wird hier deutlich, dass der Verschlusskörper 12 die Form eines flachen Zylinders hat und die Öffnung 14 durch enges Anliegen an der sich in diesem Bereich in Richtung der Eintrittsöffnung 15 verjüngenden Wand 17 des Kanals 13 verschließt. Die Pfeile 18 symbolisieren hier die Strömungsrichtung der Flüssigkeit bzw. des Kulturmediums im Kanal 13.

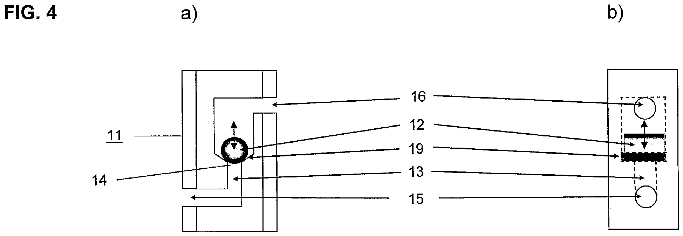

Die Figuren 4 bis 8 zeigen jeweils Schnitte durch verschiedene alternative Ausführungsformen von erfindungsgemäßen Verbindungselementen in Seiten- und Vorderansicht. Aus Gründen der Übersichtlichkeit sind die einzelnen Bestandteile der Verbindungselemente und des ventilartigen Mechanismus' in den Figuren 4 bis 8 mit den gleichen Bezugsziffern versehen wie die entsprechenden Bestandteile in den Figuren 2 und 3. Die Auflagefläche 19 für den Verschlusskörper 12 ist dabei jeweils grau unterlegt dargestellt.

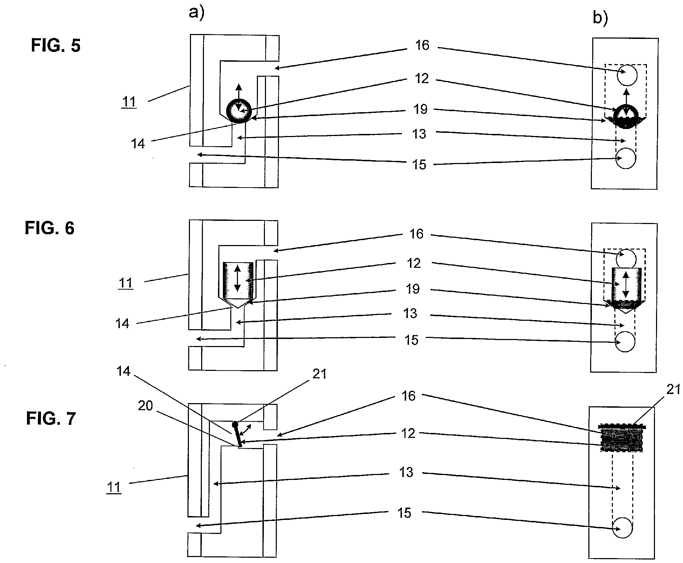

Figur 4 zeigt das Verbindungselement 11 mit dem Verschlusskörper 12 gemäß den Figuren 2 und 3. Figur 5 zeigt ein Verbindungselement 11 mit kugelförmigem Verschlusskörper 12, während Figur 6 ein Verbindungselement 11 mit kegelförmigem Verschlusskörper 12 zeigt. Die in den Figuren 2 bis 6 dargestellten Ausführungsformen haben gemeinsam, dass der Kanal 13 sich im Bereich der Öffnung 14 in Richtung der Eintrittsöffnung 15 verengt, so dass eine Auflagefläche 19 entsteht, an der der Verschlusskörper 12 anliegen kann, um die Öffnung 14 zu verschließen. Figur 7 zeigt eine weitere alternative Ausführungsform des Verbindungselements 11, bei der der Verschlusskörper 12 in Form einer Klappe ausgebildet ist. Bei dieser Ausführungsform weist der Kanal 13 in der Nähe der Austrittsöffnung 16 eine Kante 20 auf, an der die Klappe anliegen kann, so dass sie in einer Position festgehalten wird, in der die Öffnung 14 verschlossen ist. Die Klappe ist in Richtung der Austrittsöffnung 16 um die Achse 21 schwenkbar.

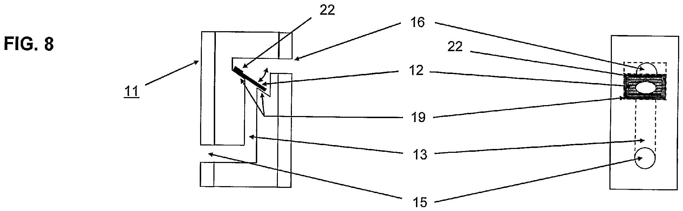

Figur 8 zeigt das Verbindungselement 11 mit einem flachen Verschlusskörper 12, wobei der Verschlusskörper 12 eine Folie ist. Die Folie ist im Befestigungsbereich 22 an dem Verbindungselement 11 befestigt, wobei es sich bei dem Befestigungsbereich 22 beispielsweise um eine Klebe- oder Schweißnaht handeln kann. Die Folie ist mit Ausnahme des Befestigungsbereichs 22 frei beweglich, kann sich also in den durch den Doppelpfeil angedeuteten Richtungen bewegen. Die Folie liegt in geschlossenem Zustand an den Auflageflächen 19 an und verschließt somit den Kanal 13. Sie kann sich aber bei Überdruck im Kanal 13 von den Auflageflächen 19 wegbewegen und so den Kanal 13 freigeben.

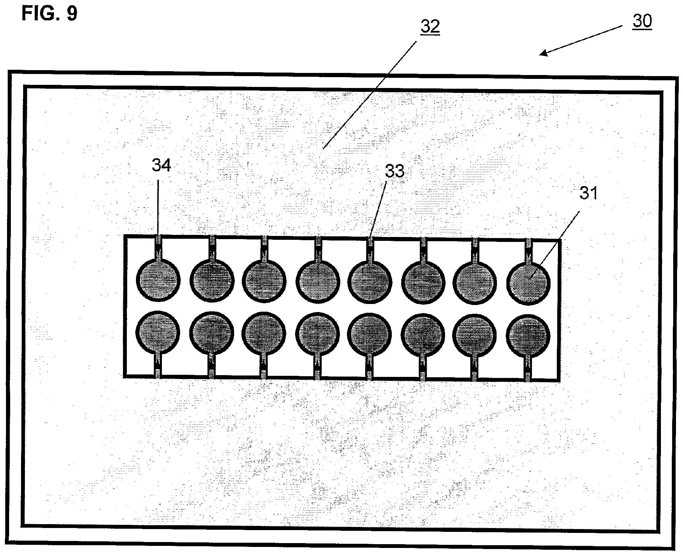

Figur 9 zeigt eine Ausführungsform der erfindungsgemäßen Vorrichtung 30 mit 16 Kulturräumen 31. Die Kulturräume 31 sind in diesem Beispiel in zwei parallelen Reihen mit je acht Kulturräumen 31 angeordnet. Jeder einzelne Kulturraum 31 ist mit einem Reservoir 32, das die Anordnung der Kulturräume 31 umgibt, über ein separates Verbindungselement 33 verbunden. Innerhalb der kanalförmigen Verbindungselemente 33 sind jeweils ventilartige Mechanismen angeordnet, die durch die Pfeile 34 symbolisiert werden. Die Funktionsweise dieser ventilartigen Mechanismen entspricht derjenigen, die zuvor bereits im Zusammenhang mit den Figuren 1 bis 8 beschrieben wurde.

### Bezugszeichenliste:

- 1: Vorrichtung
- 2: Reservoir
- 3: Kulturraum
- 4: Verbindungselement
- 5: Flüssigkeit
- 6: Zellen
- 7: Deckel
- 8 9 10: Vorrichtung
- 11: Verbindungselement
- 12: Verschlusskörper
- 13: Kanal
- 14: Öffnung
- 15: Eintrittsöffnung
- 16: Austrittsöffnung
- 17: Wand
- 18: Pfeile
- 19: Auflagefläche
- 20: Kante
- 21: Achse
- 22: Befestigungsbereich
- 30: Vorrichtung
- 31: Kulturräume
- 32: Reservoir
- 33: Verbindungselement
- 34: Pfeile

## Patentansprüche

1. Verfahren zur Kultivierung lebender Zellen in einer Flüssigkeit, wobei die Zellen in mindestens einen Kulturraum eingebracht werden, der mit mindestens einem Reservoir für die Flüssigkeit in Verbindung steht, wobei das Volumen der Flüssigkeit (5) in mindestens einem Kulturraum (3, 31) konstant gehalten wird, **dadurch gekennzeichnet, dass** das Volumen der Flüssigkeit (5) über einen ventilartigen Mechanismus konstant gehalten wird, welcher gleichzeitig einen Übertritt von Flüssigkeit (5) und/oder in der Flüssigkeit (5) gelösten oder suspendierten Substanzen aus dem Kulturraum (3, 31) in das Reservoir (2, 32) verhindert.

2. Verfahren nach Anspruch 1, **dadurch gekennzeichnet, dass** innerhalb des ventilartigen Mechanismus' zumindest ein Verschlusskörper (12) aufgrund seines Eigengewichts eine Öffnung (14) und/oder einen Kanal (13) verschließt.

3. Verfahren nach Anspruch 1 oder 2, **dadurch gekennzeichnet, dass** das Reservoir (2, 32) in unmittelbarer Nähe des Kulturraums (3, 31) angeordnet wird.

4. Verfahren nach einem der Ansprüche 1 bis 3, **dadurch gekennzeichnet, dass** der Kulturraum (3, 31) mit einem geringen Volumen Flüssigkeit (5) gefüllt wird, vorzugsweise maximal 200 µl, besonders bevorzugt maximal 100 µl oder 50 µl.

5. Verfahren nach einem der Ansprüche 1 bis 4, **dadurch gekennzeichnet, dass** das Reservoir (2, 32) mit einem Volumen gefüllt wird, das mindestens das 5-fache, vorzugsweise 10-fache, besonders bevorzugt 100-fache, des Volumens der Flüssigkeit (5) im Kulturraum (3, 31) beträgt.

6. Vorrichtung (1, 10, 30) zur Kultivierung lebender Zellen in einer Flüssigkeit, die mindestens einen Kulturraum umfasst, der über mindestens ein Verbindungselement mit mindestens einem Reservoir zur Aufnahme der Flüssigkeit verbunden ist, wobei das Verbindungselement (4, 11, 33) derart ausgebildet ist, dass das Volumen der Flüssigkeit (5) in mindestens einem Kulturraum (3, 31) konstant gehalten wird, **dadurch gekennzeichnet, dass** das Verbindungselement (4, 11, 33) einen ventilartigen Mechanismus umfasst.

7. Vorrichtung nach Anspruch 6, **dadurch gekennzeichnet, dass** das Verbindungselement (4, 11, 33) mindestens eine Öffnung (14) und/oder mindestens einen Kanal (13) umfasst.

8. Vorrichtung nach Anspruch 6 oder 7, **dadurch gekennzeichnet, dass** der ventilartige Mechanismus zumindest einen Verschlusskörper (12) umfasst.

9. Vorrichtung nach Anspruch 8, **dadurch gekennzeichnet, dass** der Verschlusskörper (12) kugel-, zylinder-, kegel- oder klappenförmig ausgebildet ist.

10. Vorrichtung nach Anspruch 8, **dadurch gekennzeichnet, dass** der Verschlusskörper (12) eine Folie ist.

11. Vorrichtung nach einem der Ansprüche 6 bis 10, **dadurch gekennzeichnet, dass** eine Vielzahl von Kulturräumen (31) vorgesehen ist, vorzugsweise 8, 16 oder 32.

12. Vorrichtung nach Anspruch 11, **dadurch gekennzeichnet, dass** die Kulturräume (31) voneinander getrennt sind, wobei jeder einzelne Kulturraum (31) ein separates Verbindungselement (33) aufweist.

13. Verwendung der Vorrichtung nach einem der Ansprüche 6 bis 12 zur Langzeit-Kultivierung von lebenden Zellen oder Geweben in geringen Mengen und/oder geringem Volumen.

14. Verwendung der Vorrichtung nach einem der Ansprüche 6 bis 12 zum Screening von Substanzen.

## Claims

1. Method for cultivating living cells in a liquid, wherein the cells are introduced into at least one culture space which is connected with at least one reservoir for the liquid, wherein the volume of the liquid (5) in at least one culture space (3, 31) is kept constant, **characterized in that** the volume of the liquid (5) is kept constant by a valve-like mechanism which concurrently prevents a transfer of liquid (5) and/or substances solved or suspended in the liquid (5) from the culture space (3, 31) into the reservoir (2, 32).

2. Method according to claim 1, **characterized in that** within the valve-like mechanism at least one sealing member (12) seals an opening (14) and/or a channel (13) due to its proper weight.

3. Method according to claim 1 or 2, **characterized in that** the reservoir (2, 32) is disposed in immediate vicinity of the culture space (3, 31).

4. Method according to any one of claims 1 to 3, **characterized in that** the culture space (3, 31) is filled with a small volume of liquid (5), preferably at most 200 µl, particularly preferred at most 100 µl or 50 µl.

5. Method according to any one of claims 1 to 4, **characterized in that** the reservoir (2, 32) is filled with a volume that is at least the 5-fold, preferably 10-fold, particularly preferred 100-fold, of the volume of the liquid (5) in the culture space (3, 31).

6. Device (1, 10, 30) for cultivating living cells in a liquid, comprising at least one culture space which is connected with at least one reservoir for receiving the liquid via at least one connecting element, wherein the connecting element (4, 11, 33) is designed such that the volume of the liquid (5) in at least one culture space (3, 31) is kept constant, **characterized in that** the connecting element (4, 11, 33) comprises a valve-like mechanism.

7. Device according to claim 6, **characterized in that** the connecting element (4, 11, 33) comprises at least one opening (14) and/or at least one channel (13).

8. Device according to claim 6 or 7, **characterized in that** the valve-like mechanism comprises at least one sealing member (12).

9. Device according to claim 8, **characterized in that** the sealing member (12) is constructed ball-, cylinder-, cone- or shutter-like.

10. Device according to claim 8, **characterized in that** the sealing member (12) is a foil.

11. Device according to any one of claims 6 to 10, **characterized in that** a plurality of culture spaces (31) is provided, preferably 8, 16 or 32.

12. Device according to claim 11, **characterized in that** the culture spaces (31) are separated from each other, wherein each single culture space (31) includes a discrete connecting element (33).

13. Use of the device according to any one of claims 6 to 12 for long-term cultivation of living cells or tissues in small amounts and/or small volume.

14. Use of the device according to any one of claims 6 to 12 for screening of substances.

## Revendications

1. Procédé consistant à cultiver des cellules vivantes dans un liquide, lors duquel on introduit les cellules dans au moins une chambre de culture qui est en liaison avec au moins un réservoir pour le liquide, le volume du liquide (5) étant maintenu à un niveau constant dans au moins une chambre de culture (3, 31), **caractérisé en ce que** le volume du liquide (5) est maintenu à un niveau constant par un mécanisme de type valve, lequel empêche simultanément un débordement de liquide (5) et/ou de substances dissoutes ou en suspension dans le liquide (5) hors de la chambre de culture (3, 31) dans le réservoir (2, 32).

2. Procédé selon la revendication 1, **caractérisé en ce qu'**à l'intérieur du mécanisme de type valve, grâce à son poids intrinsèque, au moins un organe d'obturation (12) ferme un orifice (14) et/ou un conduit (13).

3. Procédé selon la revendication 1 ou 2, **caractérisé en ce que** le réservoir (2, 32) est placé à proximité directe de la chambre de culture (3, 31).

4. Procédé selon l'une quelconque des revendications 1 à 3, **caractérisé en ce qu'**on remplit la chambre de culture (3, 31) d'un faible volume de liquide (5), de préférence d'un maximum de 200 µl, de manière particulièrement préférentielle, d'un maximum de 100 µl ou de 50 µl.

5. Procédé selon l'une quelconque des revendications 1 à 4, **caractérisé en ce qu'**on remplit le réservoir (2, 32) d'un volume qui s'élève à au moins 5 fois, de préférence à au moins 10 fois, de manière particulièrement préférentielle 100 fois le volume du liquide (5) dans la chambre de culture (3, 31).

6. Dispositif (1, 10, 30) destiné à cultiver des cellules dans un liquide, qui comprend au moins une chambre de culture, qui par l'intermédiaire d'au moins un élément de liaison est reliée avec au moins un réservoir destiné à recevoir le liquide, l'élément de liaison (4, 11, 33) étant conçu de telle sorte que le volume du liquide (5) soit maintenu à un niveau constant dans au moins une chambre de culture (3, 31), **caractérisé en ce que** l'élément de liaison (4, 11, 33) comprend un mécanisme de type valve.

7. Dispositif selon la revendication 6, **caractérisé en ce que** l'élément de liaison (4, 11, 33) comprend au moins un orifice (14) et/ou au moins un conduit (13).

8. Dispositif selon la revendication 6 ou 7, **caractérisé en ce que** le mécanisme de type valve comprend au moins un organe d'obturation (12).

9. Dispositif selon la revendication 8, **caractérisé en ce que** l'organe d'obturation (12) est conçu de forme sphérique, cylindrique, conique ou en forme de clapet.

10. Dispositif selon la revendication 8, **caractérisé en ce que** l'organe d'obturation (12) est un film.

11. Dispositif selon l'une quelconque des revendications 6 à 10, **caractérisé en ce qu'**il est prévu une pluralité de chambres de culture (31), de préférence 8, 16 ou 32.

12. Dispositif selon la revendication 11, **caractérisé en ce que** les chambres de cultures (31) sont séparées les unes des autres, chaque chambre de culture (31) individuelle comprenant un élément de liaison (33) séparé.

13. Utilisation du dispositif selon l'une quelconque des revendications 6 à 12 pour la culture à long terme de cellules vivantes ou de tissus vivants dans de faibles quantités et/ou de faibles volumes.

14. Utilisation du dispositif selon l'une quelconque des revendications 6 à 12 pour le criblage de substances.
